# EUROPEAN PATENT APPLICATION

(11) **EP 2 689 769 A1**
(43) Date of publication of application: **29.01.2014**
(21) Application number: 12760163.1
(22) Date of filing: 13.03.2012
(51) Int. Cl.: A61K 8/19, A61K 8/27, A61K 8/28, A61K 8/29, A61Q 17/04

(54) **ULTRAVIOLET ABSORBENT AND COSMETIC PREPARATION USING SAME**

(30) Priority: 23.03.2011 JP 2011063542
(71) Applicant: IHI Corporation, Tokyo 135-8710 (JP)
(72) Inventor: EGUCHI, Haruki, Tokyo 135-8710 (JP); FUCHIGAMI, Kenji, Tokyo 135-8710 (JP)
(74) Representative: Moore, Graeme Patrick
(86) International application number: PCT/JP2012/056459
(87) International publication number: WO 2012/128131

(57) **Abstract**

There are provided: an ultraviolet light absorber that has a sufficient UV-A and UV-B shielding effect, can be colored naturally to suit the color of the skin when applied to the skin, and can be favorably applied even to races with dark skin colors; and a cosmetic material using such an ultraviolet light absorber. The ultraviolet light absorber is one produced by substituting, at 10% or less, at least one element selected from the group consisting of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, Hg, La, and In into either A or B, or both of them, in a compound expressed by a general formula ABO₄ (where A represents In, Bi, Ga, or Gd and B represents Ta, Nb, or V).

## Description

### [Technical Field]

The present invention relates to a cosmetic material having ultraviolet absorbing property and a method for producing the cosmetic material.

### [Background Art]

It has been conventionally known that ultraviolet light causes various changes to the skin. Dermatologically, working wavelengths of the ultraviolet light are classified into long-wavelength ultraviolet light of 400 nm to 320nm, mid-wavelength ultraviolet light of 320 nm to 290 nm, and short wavelength ultraviolet light of 290 nm or lower and these groups of light are called UV-A, UV-B, and UV-C, respectively.

Normally, most of the ultraviolet light to which humans are exposed is sunlight; and of such ultraviolet light, ultraviolet light that reaches the ground is the UV-A and UV-B, while the UV-C hardly reaches the ground because the UV-C is absorbed in an ozone layer. If the skin is exposed to a certain light quantity or more of the UV-A and UV-B of the ultraviolet light which reaches the ground, that causes erythema or blisters, also promotes the formation of melanin, and causes changes such as occurrence of pigmentation. Therefore, it is very important to protect the skin against the UV-A and UV-B in terms of the prevention of accelerated skin senescence and the prevention of occurrence of blemishes and freckles. In view of the above, various UV-A and UV-B absorbers have been developed.

Dibenzoyl-methane derivatives are known as existing UV-A absorbers. (For example, see Patent Literature 1).

Furthermore, PABA derivatives, cinnamic acid derivatives, salicylic acid derivatives, camphor derivatives, urocanic acid derivatives, benzophenone derivatives, and heterocyle derivatives are known as UV-B absorbers. (For example, see Patent Literature 2).

These UV-A and UV-B absorbers are blended with skin drugs for external treatment such as cosmetic materials and quasi-pharmaceutical products and then used.

On the other hand, fine-particle titanium oxide is blended with, for examples, cosmetic materials intended for UV protection by utilizing ultraviolet light shielding power of the fine-particle titanium oxide; however, its protection effect is weaker than that of organic ultraviolet light absorbers used for the same purpose and a large blending quantity is required to have a high ultraviolet light protection effect. Accordingly, if an ultraviolet light protection cosmetic material in which the fine-particle titanium oxide conventionally sold in the market is blended is applied to the skin, there is fear, for example, that unnatural paleness will occur. Also, the conventional fine-particle titanium oxide shields the wavelength of the UV-B area (320 nm to 290nm), but does not shield the UV-A area (400 nm to 320nm) sufficiently.

So, for example, iron-containing ultrafine-particle rutile-type titanium oxide and iron-containing titanium dioxide, and so on are suggested as titanium oxides which have superior ultraviolet light shielding effects and do not result in unnatural whiteness. (For example, see Patent Literature 3 and 4).

Moreover, titanium oxide - ceric oxide composite sol is also suggested as a combination of titanium oxide and ceric oxide. (For example, see Patent Literature 5).

Furthermore, a combination of a lower-level titanium oxide pigment, titanium oxide, and cerium oxide is also suggested. (For example, see Patent Literature 6).

Furthermore, an ultraviolet light absorber made of a silicon cluster or a germanium cluster is also suggested. (For example, see Patent Literature 7).

A cosmetic material that has UV-A and UV-B shielding effects and will not color a cosmetic material even when mixed into the cosmetic material is introduced. (For example, see Patent Literature 8).

Then, an ultraviolet light absorber, which contains at least one type of silicon oligomer with n being 2 to 5 in the following formula (I), and a skin drug for external treatment, in which the above-described ultraviolet light absorber is combined, are introduced. (For example, see Patent Literature 9).

Formula (I) - (Si)ₙ-N<

### [Citation List]

### [Patent Literature]

[Patent Literature 1] Japanese Patent Application Laid-Open (Kokai) Publication No. 05-247063
[Patent Literature 2] Japanese Patent Application Laid-Open (Kokai) Publication No. 2003-212711
[Patent Literature 3] Japanese Patent Application Laid-Open (Kokai) Publication No. 05-330825
[Patent Literature 4] Japanese Patent Application Laid-Open (Kokai) Publication No. 07-69636
[Patent Literature 5] Japanese Examined Patent Publication (Kokoku) No. 06-650
[Patent Literature 6] Japanese Patent Application Laid-Open (Kokai) Publication No. 60-245671
[Patent Literature 7] Japanese Patent Application Laid-Open (Kokai) Publication No. 2005-314408
[Patent Literature 8] Japanese Patent Application Laid-Open (Kokai) Publication No. 11-180829
[Patent Literature 9] Japanese Patent Application Laid-Open (Kokai) Publication No. 2006-27917

### [Summary of Invention]

### [Problems to be Solved by the Invention]

However, iron-containing titanium oxide is superior in prevention of a non-powdery finish caused by the titanium oxide and in the UV-A area shielding property, but its red color because of the iron oxide is so strong that it is difficult to adjust the color tone. The iron-containing titanium oxide becomes orange when blended with cosmetic materials and its color tone is closer to a foundation than to a general milky lotion. If the iron-containing titanium oxide is used as a cosmetic foundation, there is fear that it will affect the color tone of the foundation or might result in a somber makeup.

Moreover, since the form of titanium oxide - ceric oxide composite sol is sol, there are limitations on blending with cosmetic materials and there is still room for improvement in terms of durability.

Furthermore, combinations of titanium oxide and cerium oxide are not actually used for cosmetic materials which are not intended for coloring, because lower-level titanium oxide is a black pigment; and an ultraviolet light absorber made of a silicon cluster or germanium cluster is not actually used for cosmetic materials which are not intended for coloring, because the silicon cluster and the germanium cluster are black pigments. Also, these black pigments do not sufficiently absorb light of the UV-A area (400 nm to 320 nm) according to their light absorption spectra.

Furthermore, the cosmetic material according to Patent Literature 8 does not cause a non-powdery finish after applied to the skin; or is not colored naturally to suit the color of the skin. Also, it cannot obtain the sufficient effect of absorbing ultraviolet light.

Moreover, in fact, the ultraviolet light absorber according to Patent Literature 9 becomes black and, therefore, is not suited for a cosmetic material.

The present invention was devised in light of the circumstances described above and it is an object of the invention to provide: an ultraviolet light absorber that has a sufficient UV-A and UV-B shielding effect, can be colored naturally to suit the color of the skin when applied to the skin, and can be favorably applied even to races with dark skin colors; and a cosmetic material using the ultraviolet light absorber.

### [Means for Solving the Problems]

In order to achieve the above-described object, the present invention provides an ultraviolet light absorber produced by substituting, at 10% or less, at least one element selected from the group consisting of Sc (scandium), Ti (titanium), V, Cr (chromium), Mn (manganese), Co (cobalt), Cu (copper), Ga, Ge (germanium), As (arsenic), Y (yttrium), Zr (zirconium), Nb, Mo (molybdenum), Tc (technetium), Ru (ruthenium), Rh (rhodium), Pd (palladium), Ag (silver), Cd (cadmium), Sn (tin), Sb (antimony), Hf (hafnium), W (tungsten), Re (rhenium), Os (osmium), Ir (iridium), Pt (platinum), Au (gold), Hg (mercury), and La (lanthanum) into either A or B, or both of them, in a compound expressed by a general formula ABO₄ (where A represents In (indium), Bi (bismuth), Ga (gallium), or Gd (gadolinium) and B represents Ta (tantalum), Nb (niobium), or V (vanadium)).

In this way, a white compound can be colored by substituting, at 10% or less, at least one element selected from the above-listed group into either A or B, or both of them, in the compound expressed by the aforementioned general formula ABO₄. Therefore, it is possible to provide an ultraviolet light absorber which has the sufficient UV-A and UV-B shielding effect and can be colored naturally to suit the color of the skin when applied to the skin.

Moreover, with the ultraviolet light absorber according to the present invention, at least one element selected from the above-listed group can be substituted at 1 % or more.

Furthermore, the present invention provides an ultraviolet light absorber produced by substituting Fe (iron) at 1% or more to 10% or less and Zn at 1% or more to 10% or less into either A or B, or both of them, in a compound expressed by the general formula ABO₄ (where A represents In, Bi, Ga, or Gd and B represents Ta, Nb, or V). This ultraviolet light absorber causes a white compound to become brown, so that it has a sufficient UV-A and UV-B shielding effect and can be colored naturally to suit the color of brown skin. Also, with this ultraviolet light absorber, Fe and Zn should more preferably be substituted at 5%, respectively.

Furthermore, the present invention provides an ultraviolet light absorber produced by substituting Fe at 1 % or more to 10% or less into either A or B, or both of them, in a compound expressed by the general formula ABO₄ (where A represents In, Bi, Ga, or Gd and B represents Ta, Nb, or V). This ultraviolet light absorber causes a white compound to become light brown, so that it has a sufficient UV-A and UV-B shielding effect and can be colored naturally to suit the color of light brown skin. Also, with this ultraviolet light absorber, Fe should more preferably be substituted at 10%.

Furthermore, the present invention provides an ultraviolet light absorber produced by substituting Zn (zinc) at 1% or more to 10% or less into either A or B, or both of them, in a compound expressed by the general formula ABO₄ (where A represents In, Bi, Ga, or Gd and B represents Ta, Nb, or V). This ultraviolet light absorber causes a white compound to become yellow, so that it has a sufficient UV-A and UV-B shielding effect and can be colored naturally to suit the color of yellow skin. Also, with this ultraviolet light absorber, Zn should more preferably be substituted at 10%.

Furthermore, the present invention provides an ultraviolet light absorber produced by substituting Ni (nickel) at 1% or more to 10% or less into either A or B, or both of them, in a compound expressed by the general formula ABO₄ (where A represents In, Bi, Ga, or Gd and B represents Ta, Nb, or V). This ultraviolet light absorber causes a white compound to become light yellow, so that it has a sufficient UV-A and UV-B shielding effect and can be colored naturally to suit the color of light yellow skin. Also, with this ultraviolet light absorber, Ni should more preferably be substituted at 10%.

Furthermore, the present invention provides an ultraviolet light absorber produced by substituting In at 1% or more to 10% or less excessively into a compound expressed by the general formula ABO₄ (where A represents In, Bi, Ga, or Gd and B represents Ta, Nb, or V). This ultraviolet light absorber causes a white compound to become yellow, so that it has a sufficient UV-A and UV-B shielding effect and can be colored naturally to suit the color of yellow skin. Also, with this ultraviolet light absorber, In should more preferably be substituted at 10% excessively.

Furthermore, the present invention can provide a cosmetic material containing the aforementioned ultraviolet light absorber.

Incidentally, examples of the compound expressed by the general formula ABO₄ (where A represents In (indium), Bi (bismuth), Ga (gallium), or Gd (gadolinium) and B represents Ta (tantalum), Nb (niobium), or V (vanadium)) include InTaO₄ (indium tantalum oxide), InNbO₄ (indium niobium oxide), BiTaO₄ (bismuth tantalum oxide), BiNbO₄ (bismuth niobium oxide), BiVO₄ (bismuth vanadium oxide), GaTaO₄ (gallium tantalum oxide), and GdTaO₄ (gadolinium tantalum oxide).

### [Advantageous Effects of Invention]

Since the ultraviolet light absorber and the cosmetic material according to the present invention can absorb UV-A and UV-B effectively, they can demonstrate a sufficient UV-A and UV-B shielding effect. Moreover, they can be colored naturally to suit the color of the skin when applied to the skin; and can be favorably applied even to races with dark skin colors.

### [Brief Description of Drawings]

[Fig. 1] Fig. 1 illustrates the relationship between doping elements (substitutable elements), which are derived based on a first principle calculation, and their bond energy according to an embodiment of the present invention.
[Fig. 2] Fig. 2 illustrates the relationship between electron energy and electron density when part of InTaO₄ is substituted with a doping element in the embodiment of the present invention.
[Fig. 3] Fig. 3 illustrates the relationship between the electron energy and the electron density when part of InTaO₄ is substituted with a doping element in the embodiment of the present invention.
[Fig. 4] Fig. 4 illustrates the relationship between the electron energy and the electron density when part of InTaO₄ is substituted with a doping element in the embodiment of the present invention.
[Fig. 5] Fig. 5 illustrates the relationship between the electron energy and the electron density when part of InTaO₄ is substituted with a doping element in the embodiment of the present invention.
[Fig. 6] Fig. 6 illustrates absorption spectra of ultraviolet light absorbers (samples) produced in Example 1 of the embodiment of the present invention by a diffuse reflectance spectroscopy method.
[Fig. 7] Fig. 7 illustrates the relationship between the electron energy and the electron density when part of InTaO₄ is substituted with a doping element in the embodiment of the present invention.
[Fig. 8] Fig. 8 illustrates absorption spectra of ultraviolet light absorbers (samples) produced in Example 2 of the embodiment of the present invention by the diffuse reflectance spectroscopy method.
[Fig. 9] Fig. 9 is a flowchart illustrating steps of producing the cosmetic material according to the present invention by a sol-gel method.

### [Description of Embodiments]

Next, an ultraviolet light absorber according to an embodiment of the present invention will be explained. Incidentally, this embodiment will describe a case where InTaO₄ is used as a compound expressed by a general formula ABO₄ (where A represents In, Bi, Ga, or Gd and B represents Ta, Nb, or V). Also, to substitute an element(s) to InTaO₄ and substitute part of either In or Ta or both of them in InTaO₄ with the element is called "dope" and an element (s) which is used to substitute at least part of InTaO₄ may be sometimes called the "doping element(s).

### <Cosmetic Material>

The ultraviolet light absorber according to the embodiment of the present invention is produced by substituting at least one element selected from the group consisting of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, Hg, La, Fe, Zn, and Ni into either In or Ta, or both of them, in InTaO₄ and substituting the part of InTaO₄ with the element (doping element). This ultraviolet light absorber may be one with only part of In of InTaO₄ substituted with the doping element or one with only part of Ta of InTaO₄ substituted with the doping element. Furthermore, the cosmetic material may be one with part of both In and Ta substituted with the doping element(s). Furthermore, there may be one type of doping element or multiple types of doping elements to substitute part of InTaO₄.

Furthermore, the ultraviolet light absorber according to the present invention is produced by excessively substituting In into InTaO₄ and substituting part of Ta with In.

### <Selection of Doping Element >

In this embodiment, a doping element is selected from a multiplicity of elements existing on earth by performing simulations based on a first principle calculation. All analysis methods used for this analysis are based on density functional formalism. Norm-conserving pseudopotential that uses Kleinman-Bylander form (L. Kleinman and D. M. Bylander, Phys. Rev. Lett. 48 (1982) 425) is used for interactions between electrons and ions. A method of Troullier and Martins (N. Troullier and J. L. Martins, Phys. Rev. B. 43 (1991) 1993) is used for a pseudo-wave function and pseudopotential; and a method of Perdew and Zunger (J. P. Perdew and A. Zunger, Phys. Rev. B. 23 (1981) 5048) is used for exchange correlation energy.

Cutoff energy by plane wave energy is set so that an error between a value of a lattice constant, which corresponds to a minimum value of entire energy when calculating the entire energy of an electronic system by using the lattice constant as a function, and an experimental value becomes 5% or less. When selecting the doping element, a method of selecting the doping element through a trial and error process by repeating sample creation and evaluation experiments. However, since it can be assumed that a very large number of doping elements capable of substituting at least part of InTaO₄, and a very large number of combinations of such doping elements may exist, the efficiency of the method of repeating sample creation and evaluation experiments when selecting the doping element is very low. So, in this embodiment, the doping element is selected by a method including mainly the following two steps (a first step and a second step).

### (First Step)

Firstly, whether molecules which are formed after doping InTaO₄, from among a large number of elements that can be considered to be available as doping elements for doping InTaO_{4,}, can exist as molecules or not (whether they are stable molecules or not) is judged based on the first principle calculation. Then, the element that can be used as the doping element is selected (screened) based on the judgment result. Whether the molecules formed after doping are stable molecules or not is judged in consideration of bond energy of the formed molecules. Specifically speaking, a permitted value of the bond energy is set in advance and then whether the bond energy of the molecules generated when at least part of InTaO₄ is substituted with a specified element becomes the permitted value or less is judged. The first principle calculation is used to derive this bond energy. Then, if the derived bond energy is equal to or less than the above-mentioned permitted value according to a simulation result based on the first principle calculation, it is determined that the relevant molecules can exist as molecules (they are stable molecule); and if the derived bond energy is more than the permitted value, it is determined that the relevant molecules cannot exist as molecules (they are unstable molecule). In other words, an element whose molecules after doping InTaO₄ become stable molecules is selected as an element capable of doping InTaO₄ (doping element) from among a large number of elements existing on earth.

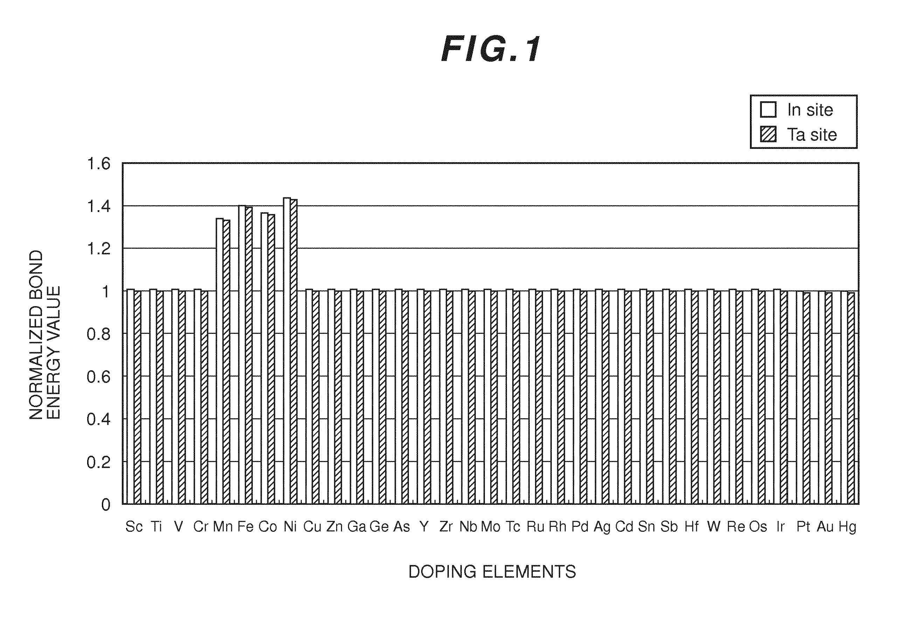

Fig. 1 illustrates the relationship between doping elements derived based on the first principle calculation, and their bond energy. The horizontal axis of a graph illustrated in Fig. 1 represents the elements for doping InTaO₄ and the vertical axis represents a value obtained by normalizing a bond energy value of molecules generated after doping InTaO₄ with the element indicated on the horizontal axis, by using a bond energy value of InTaO₄ which is not doped. Incidentally, These bond energy values are derived based on the first principle calculation. Moreover, data indicated as "In site" in Fig. 1 are simulation results when In atoms of InTaO₄ were substituted with the doping element; and data indicated as "Ta site" are simulation results when Ta atoms of InTaO₄ were substituted with the doping element.

Fig. 1 shows that when any element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg is used to dope InTaO₄, its bond energy is equivalent to or higher than that of InTaO₄ which is a starting material (InTaO₄ which is not doped). Fig. 1 also shows that there is no large difference in the bond energy when the In atoms of InTaO₄ were substituted with the doping element and when the Ta atoms were substituted with the doping element. When energy values were derived with respect to La and In in the same manner based on the first principle calculation, it was acknowledged that La and In have bond energy equivalent to or more than that of InTaO₄ which is the starting material (InTaO₄ which is not doped). Accordingly, it is acknowledged that even when InTaO₄ is doped with each of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, Hg, La, and In, the molecules formed after doping become stable molecules and these elements are the doping elements capable of doping InTaO₄.

### (Second Step)

Next, regarding each of the doping elements which are capable of doping InTaO₄ and selected in the first step, whether molecules formed after doping InTaO₄ with the relevant doping element can be effective as an ultraviolet light absorber is judged based on the first principle calculation. Then, the doping element which can be used as the ultraviolet light absorber is selected based on that judgment result. Specifically speaking, when a certain doping element from among the plurality of doping elements selected in the first step is used to dope InTaO₄, whether molecules formed after that doping absorb the ultraviolet light or not is selected in consideration of a band gap. Incidentally, the first principle calculation is also used for this selection.

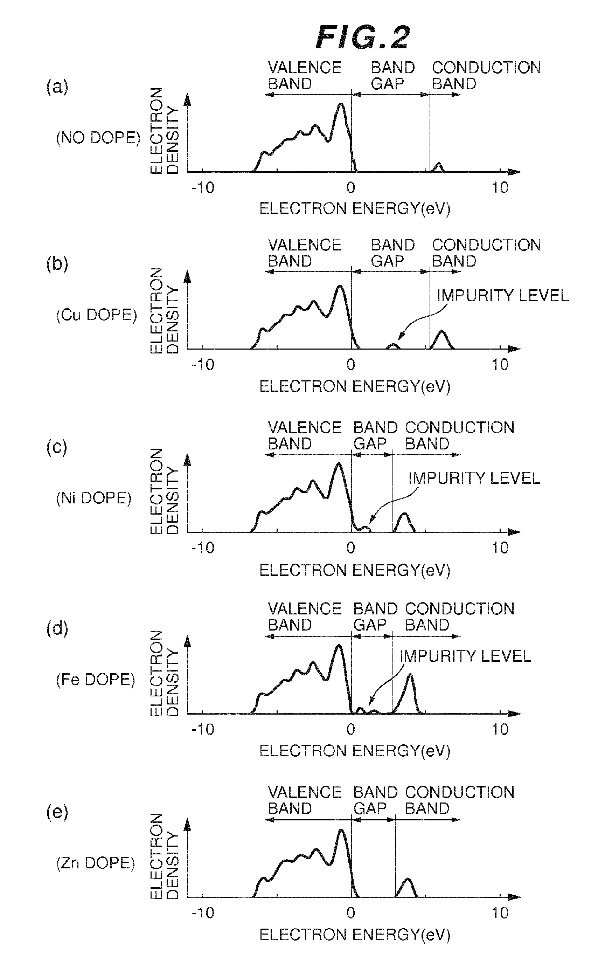

Fig. 2 to Fig. 5 illustrate the relationship between electron energy and electron density when part of InTaO₄ is substituted with the doping element. With a graph in each of Fig. 2 to Fig. 5, the horizontal axis represents the electron energy and the vertical axis represents the electron density.

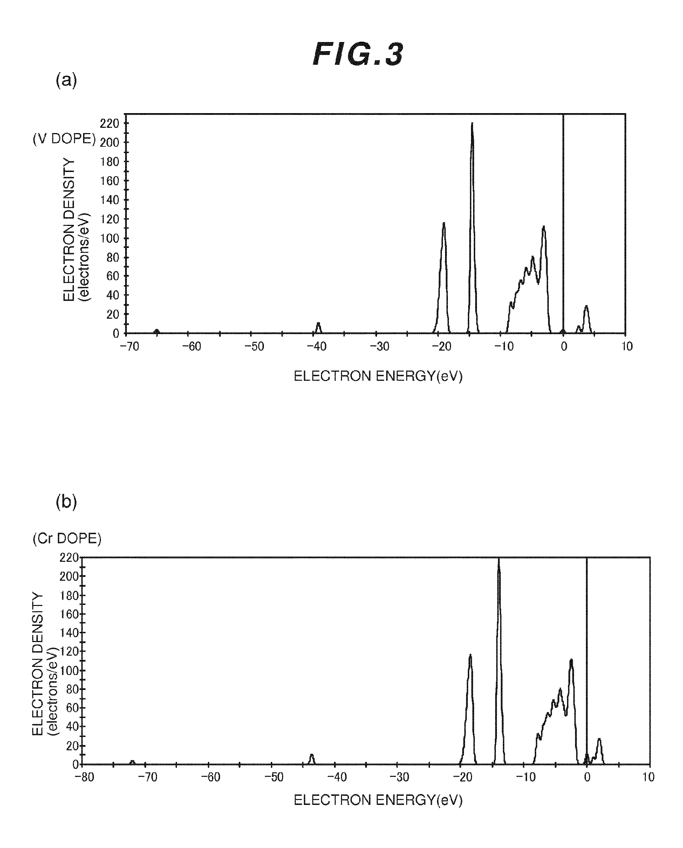

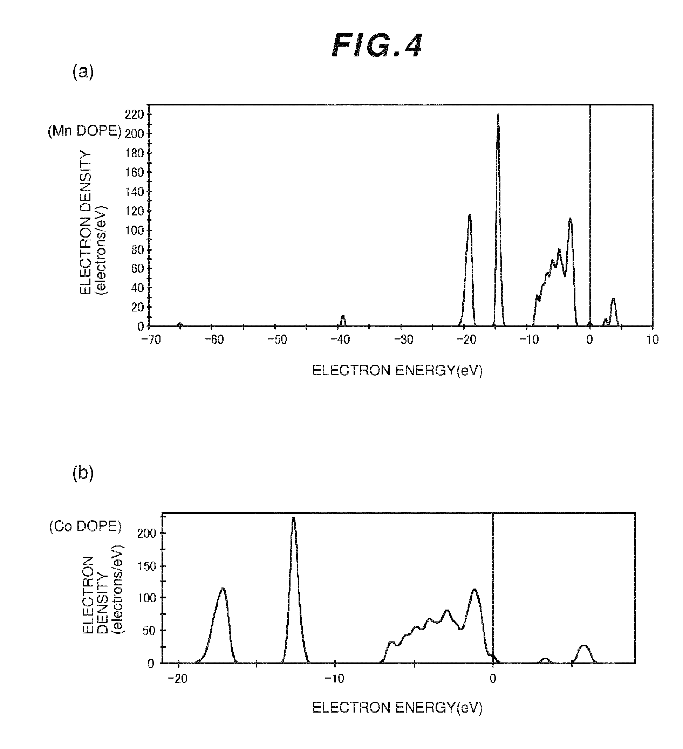

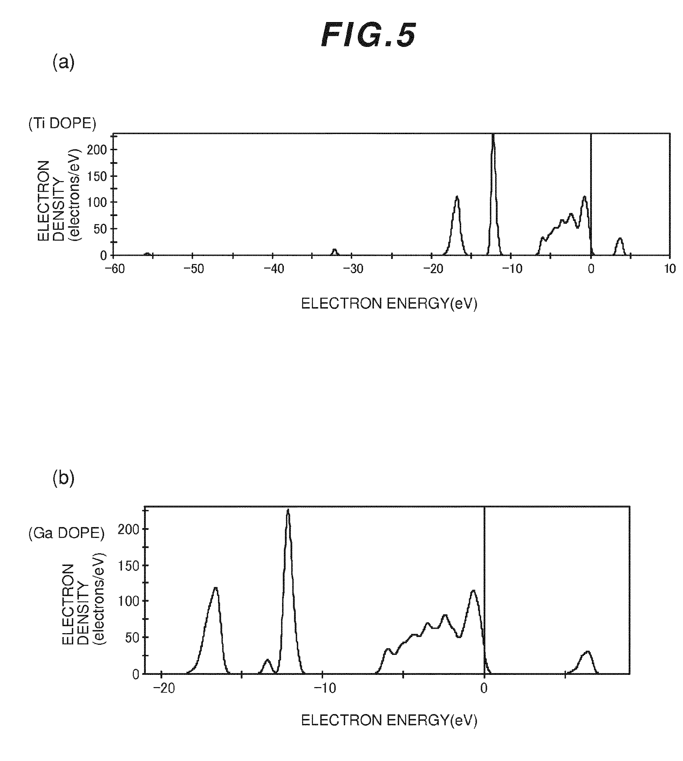

Specifically, Fig. 2(a) indicates data about InTaO₄ which was not doped with the doping element; and Fig. 2(b) to Fig. 2(e) indicate data when 6.25% In of InTaO₄ was substituted with Cu, Ni, Fe, and Zn, respectively. Moreover, Fig. 3(a) and Fig. 3(b) indicate data when 1% In in InTaO₄ was substituted with V and Cr, respectively. Furthermore, Fig. 4(a) and Fig. 4(b) indicate data when 1% In was substituted with Mn and Co, respectively. Furthermore, Fig. 5(a) and Fig. 5(b) indicate data when 1% In was substituted with Ti and Ga, respectively.

Fig. 2(c) to Fig. 2(e) show that a conduction band shifts to a lower energy side and a band gap reduces by doping InTaO₄ with Ni, Fe, and Zn, respectively. Moreover, Fig. 2(c) to Fig. 2(e) also show that the electron density of the conduction band has increased. As a result, it is found that efficient optical absorption can be performed by doping InTaO₄ with Ni, Fe, and Zn, respectively.

Furthermore, Fig. 2(e) shows that when the doping element is Zn, a new electron level (impurity level) is not formed near a valence band. Specifically speaking, it shows that when the doping element is Zn, the band gap reduces by doping the doping element and the impurity level is not formed; and, therefore, optical absorption can be performed with better efficiency. Furthermore, Fig. 5(a) and Fig. 5(b) show that the same results as in the case of Zn are obtained when Ti, Ga, or the like is used as the doping element.

As a result, Fig. 2 to Fig. 5 show that, for example, the size of the band gap, which affects light absorption property, and the electron density change considerably depending on the type of the doping element used to substitute part of InTaO₄. Then, Fig. 2 to Fig. 5 also show that each of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, Hg, La, and In can be used as the doping element in order to absorb the ultraviolet light; and particularly, it is desirable to use Ni, Fe, Zn, Ti, or Ga as the doping element for the ultraviolet light absorber; and of the above-listed doping elements, Zn, Ti, or Ga should preferably be used.

### (Example 1)

Experiments were conducted with respect to Zn, Fe, and Ni from among the plurality of doping elements selected based on the first principle calculation including the first step and the second step as described earlier. Specifically speaking, experiment objects were prepared by doping InTaO₄ with Zn, Fe, and Ni, respectively and the experiments described below were conducted. Furthermore, the same experiments were also conducted with respect to InTaO₄ and TiO₂ which were not doped as a comparison.

An ultraviolet light absorber according to this embodiment can be synthesized by a normal solid reaction method, that is, by mixing respective metal components, which are raw materials, at target composition rates and then burning the obtained mixture in air under normal atmospheric pressure. Moreover, various methods such as various sol-gel methods and complex polymerization methods using metal alkoxides and metal salts as raw materials can be used. In this case, the solid reaction method was used to create samples of the ultraviolet light absorber. The amount of the doping element was set so that 10% substitution would be performed relative to In or Ta; and the mixture was burnt at 1150 degrees Celsius for 48 hours.

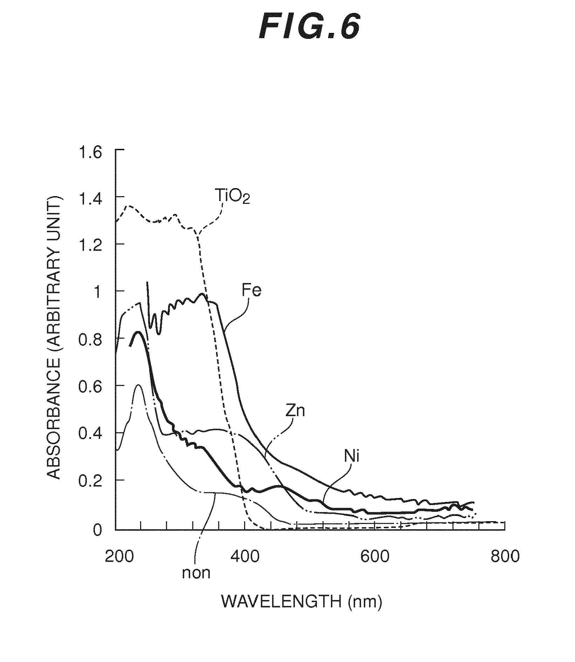

Fig. 6 illustrates absorption spectra of the ultraviolet light absorbers (samples) produced by the diffuse reflectance spectroscopy method in Example 1. Fig. 6 shows that when Fe is used as the doping element, the ultraviolet light absorber exhibits particularly large absorbance in the ultraviolet light area. Incidentally, regarding the shape of the ultraviolet light absorber according to the present invention, it is desirable that it is composed of fine particles and has a large surface area in order to effectively absorb light. An oxide prepared by the solid reaction method has large particles and a small surface area; however, the particle size can be reduced by grinding the particles with, for example, a ball mill. Moreover, the fine particles can be molded into a desired shape and used.

Moreover, referring to Fig. 6, "non" indicates a sample of InTaO₄ which was not doped with the doping element; "TiO₂" indicates a sample of titanium oxide with anatase structure; "Fe" indicates a sample in which Fe was substituted at 10% as a substitution element into InTaO₄; "Zn" indicates a sample in which Zn was substituted at 10% as a substitution element into InTaO₄; and "Ni" indicates a sample in which Ni was substituted at 10% as a substitution element into InTaO₄.

Fig. 6 shows that "non" and "TiO₂" absorbed light of a wavelength of 400 nm or less, but hardly absorbed light of a wavelength of 400 nm or more. On the other hand, Fig. 6 shows that "Fe," "Zn," and "Ni" absorbed both the light of the wavelength of 400 nm or less and the light of the wavelength of 400 nm or more which causes coloring. Incidentally, "Fe" was light brown, "Zn" was yellow, and "Ni" was light yellow.

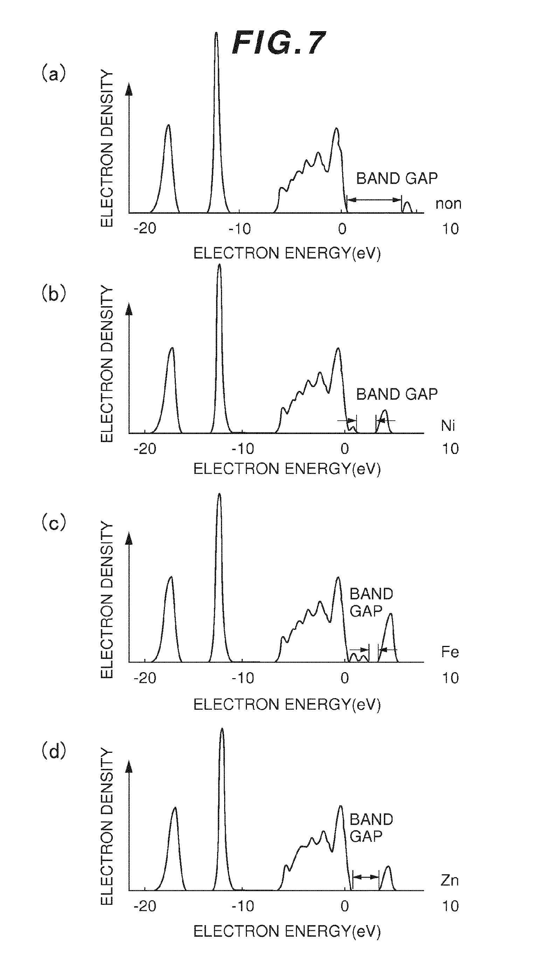

Furthermore, Fig. 7 illustrates the relationship between electron state density and electron energy by the first principle calculation corresponding to "non," "Ni," "Fe," and "Zn." Since the doping element is not substituted into InTaO₄ in the case of "non," "non" has a wide band gap; however, in the cases of "Ni," "Fe," and "Zn," Fig. 7 shows that their band gaps are narrow as a result of coloring of the samples.

### (Example 2)

Next, experiments were conducted by using experiment objects produced by doping InTaO₄ with Mo and La, respectively, doping InTaO₄ with Zn and Fe, doping InTaO₄ with Zn and Fe, and doping InTaO₄ with Zn and In by the same method used as in Example 1. Also, the same experiment was conducted on InTaO₄ which was not doped, as a comparison. Incidentally, the doping element was substituted at 10%.

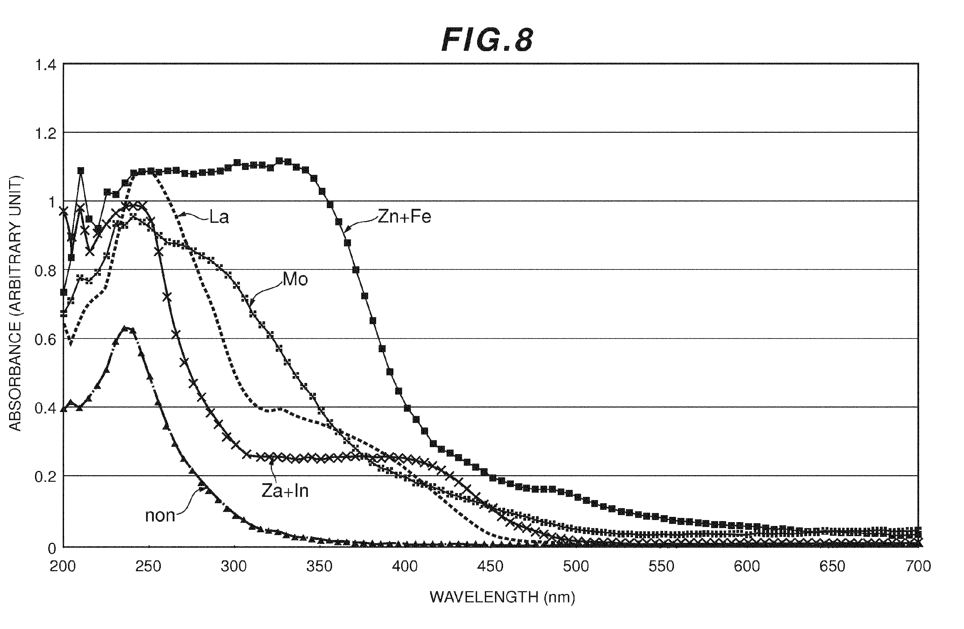

Fig. 8 illustrates absorption spectra of ultraviolet light absorbers (samples) produced in Example 2 by the diffuse reflectance spectroscopy method. Referring to Fig. 8, "non" indicates a sample of InTaO₄ which was not doped with the doping element; "Mo" indicates a sample in which Mo was substituted at 10% as a substitution element into InTaO₄; "La" indicates a sample in which La was substituted at 10% as a substitution element into InTaO₄; "Zn+Fe" indicates a sample in which Zn and Fe were substituted at 5%, respectively, as substitution elements into InTaO₄; and "Zn+In" indicates a sample in which Zn and In were substituted at 5%, respectively, as substitution elements into InTaO₄.

Fig. 8 shows that "non" absorbed the light of the wavelength of 400 nm or less, but hardly absorbed the light of the wavelength of 400 nm or more. On the other hand, Fig. 6 shows that "Mo," "La," "Zn+Fe," and "Zn+In" absorbed both the light of the wavelength of 400 nm or less and the light of the wavelength of 400 nm or more. Incidentally, "Mo" was light brown, "La" was gray, "Zn+Fe" was gray, and "Zn+In" was light gray.

Furthermore, an experiment was conducted by using an experiment object produced by doping InTaO₄ with In by the same method as in Example 1 and Example 2. As a result, it was found that the obtained sample absorbed both the light of the wavelength of 400 nm or less and the light of the wavelength of 400 nm or more. Incidentally, this sample was light yellow.

Next, a blending example of sun-block cream is shown below as an example of a cosmetic material using a sample of the ultraviolet light absorber according to the present invention, in which Fe is substituted at 10% as a substitution element into InTaO₄.

### <Blending Example of Sun-block Cream >

cetanol 1.0 wt%
stearic acid 2.0 wt%
cholesterol 1.0 wt%
squalene 5.0 wt%
jojoba oil 4.0 wt%
octyl paramethoxy cinnamate 4.0 wt%
polyethoxylated (40) hydrogenated castor oil 1.0 wt%
sorbitan monostearate 2.0 wt%
product of the present invention 7.0 wt%
butylparaben 0.1 wt%
methylparaben 0.1 wt%
ethanol 3.0 wt%
glycerin 10.0 wt%
cow placenta extract 1.0 wt%
perfume material 0.05 wt%
purified water balance

This cosmetic material absorbed both the light of the wavelength of 400 nm or less and the light of the wavelength of 400 nm or more and has a sufficient UV-A and UV-B shielding effect as sun-block cream; and when the cosmetic material was used on races with relatively dark skin colors, it was colored naturally so as to suit the color of the skin.

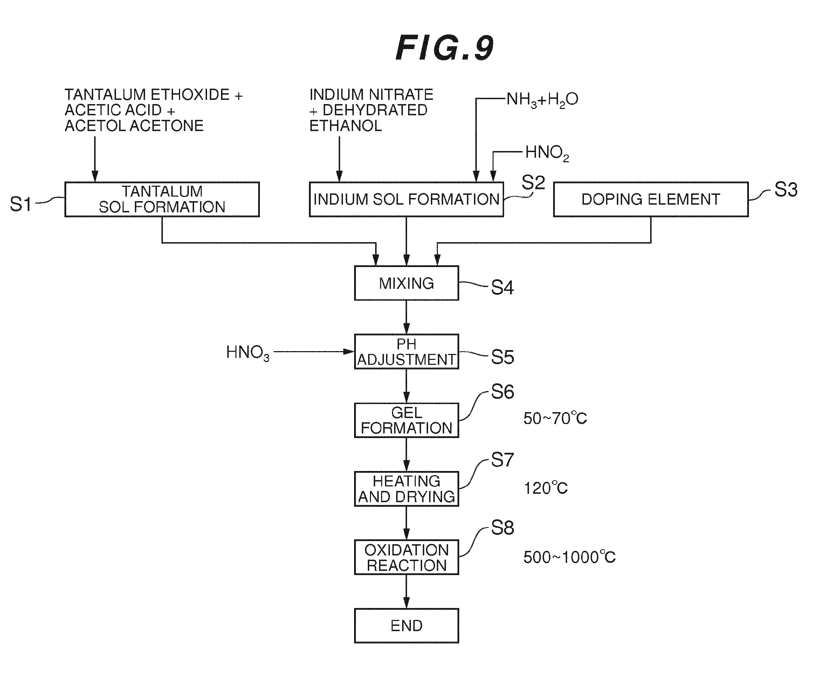

Incidentally, the ultraviolet light absorber according to this embodiment can also be produced by various sol-gel methods as mentioned above. Examples of the sol-gel methods can include a method including tantalum sol formation step S1, indium sol formation step S2, doping element dope step S3, mixed liquid formation step S4, PH adjustment step S5, gel formation step S6, heating and drying step S7, and oxidation reaction step S8 as shown in Fig. 9.

In the tantalum sol formation step S1, tantalum sol containing fine particles of tantalum in a colloidal state is synthesized by a chemical reaction. In this tantalum sol synthesis step S1, for example, tantalum ethoxide (Ta(OC₂H₅)₅), tantalum methoxide (Ta(OCH₃)₅), or tantalum alkoxide (Ta(OCₙH₂ₙ₊₁)₅, n=1 to 6) is used as a tantalum particle source. Moreover, in this tantalum sol formation step S1, 0 to 10 mol of acetyl acetone and 0 to 10 mol of acetic acid are mixed with 1 mol of tantalum. More specifically, firstly tantalum oxide sol (0.005 mol of tantalum ethoxide + 10 ml of acetic acid + 10 ml of acetyl acetone) is prepared by stirring it with a stirrer overnight.

Next, in the indium sol formation step S2, indium sol containing fine particles of indium in a colloidal state is synthesized by a chemical reaction. In this indium sol formation step S2, indium nitrate (In(NO₃)₃·nH₂O, n=0 to 6) or indium alkoxide (In(OCₙH₂ₙ₊₁)₃, n=1 to 6) is used as an indium particle source. Moreover, in this indium sol formation step S2, 0.1 to 10 mol of anhydrous ethanol, 0.1 to 10 mol of nitric acid, and 0.1 to 10 mol of ammonium are mixed with 1 mol of indium. More specifically, a mixture of 0.005 mol of indium nitrate + 20 ml of anhydrous ethanol is prepared; and then 1.5 ml of 25% NH₃-H₂O (approximately 0.01 mol) is slowly added to the above-obtained mixture while being stirred; and a HNO₃ solution (approximately 0.2 ml) is added to the above-obtained mixture, thereby synthesizing transparent sol.

Next, in the doping element dope step S3, the sol obtained above is substituted with at least one element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, Hg, La, and In.

Then, in the mixed liquid formation step S4, the tantalum sol, the indium sol, and the doping element are mixed, thereby forming a mixed liquid.

Subsequently, in the PH adjustment step S5, nitric acid is added as appropriate to the mixed liquid obtained in the mixed liquid formation step S4, thereby adjusting PH of the mixed liquid to become 6 to 7.

Next, in the gel formation step S6, the mixed liquid obtained in the PH adjustment step S5 is stirred with a stirrer overnight while maintaining the temperature of the mixed liquid at 50 to 70 degrees Celsius, thereby allowing the mixed liquid to gradually evaporate and enter a gel state.

Then, in the heating and drying step S7, the precursor gel obtained in the gel formation step S6 is further dried overnight at 120 degrees Celsius.

Subsequently, in the oxidation reaction step S8, an oxidation reaction of the gel dried in the heating and drying step S7 is performed at a temperature within the range from 500 to 1000 degrees Celsius, thereby forming solid InTaO₄. As a result, the ultraviolet light absorber is obtained.

Accordingly, a specific surface area of particles can be increased by atomizing the particles by the sol-gel method by using the tantalum sol, which contains the tantalum particles in the colloidal state, the indium sol, which contains the fine particles of indium in the colloidal state, and the doping element; and, therefore, the cosmetic material of nano-order grain size can be produced (synthesized). The cosmetic material obtained by using the sol-gel method can also absorb the ultraviolet light well. Moreover, the grain size of this cosmetic material is constant; and when this cosmetic material is applied to (or is fitted tightly to) the bare skin, it also has the effects of making the skin look fine-textured, transparent, natural, and beautiful.

Incidentally, the ultraviolet light absorber according to the present invention can produce color tones corresponding to skin colors of all races by selecting the doping element and its substitution rate (introduction rate). For example, it is possible to produce color tones close to ocher, medium brown, or black by substituting Fe within the range of 1 % to 10% into (or with) either In or Ta, or both of them, in InTaO₄.

Moreover, this embodiment has described the case where samples in which Cu, Ni, Fe, Zn, V, Cr, Ti, Ga, Mn, Co, Mo, La, Zn+Fe, and Zn+In were substituted into InTaO₄ were assessed; and when samples produced by substituting at least one element selected from the group consisting of Sc, Ge, As, Y, Zr, Nb, Tc, Ru, Rh, Pd, Ag, Cd, Sn, Sb, Hf, W, Re, Os, Ir, Pt, Au, Hg, and In within the range from 1% to 10% were assessed in the same manner, the samples absorbed both the light of the wavelength of 400 nm or less and the light of the wavelength of 400 nm or more and these samples were colored depending on the type of a doping element and its blending quantity.

Furthermore, this embodiment has described the case where InTaO₄ is used as the compound expressed by the general formula ABO₄; however, the invention is not limited to this example. When, for example, InNbO₄, BiTaO₄, BiNbO₄, BiVO₄, GaTaO₄, or GdTaO₄ was used as the compound expressed by the general formula ABO₄ and samples were produced by substituting at least one element selected from the group consisting of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, Hg, La, and In within the range from 1% to 10% into each of the above-listed compounds and the obtained samples were assessed in the same manner, the samples absorbed both the light of the wavelength of 400 nm or less and the light of the wavelength of 400 nm or more and these samples were colored depending on the type of the doping element and its blending quantity.

## Claims

1. An ultraviolet light absorber produced by substituting, at 10% or less, at least one element selected from the group consisting of Sc, Ti, V, Cr, Mn, Co, Cu, Ga, Ge, As, Y, Zr, Nb, Mo, Tc, Ru, Rh, Pd, Ag, Cd, Sn, Sb, Hf, W, Re, Os, Ir, Pt, Au, Hg, and La into either A or B, or both of them, in a compound expressed by a general formula ABO₄ (where A represents In, Bi, Ga, or Gd and B represents Ta, Nb, or V).

2. The ultraviolet light absorber according to claim 1, wherein at least one element selected from the above-listed group can be substituted at 1% or more.

3. An ultraviolet light absorber produced by substituting Fe at 1% or more to 10% or less and Zn at 1 % or more to 10% or less into either A or B, or both of them, in a compound expressed by a general formula ABO₄ (where A represents In, Bi, Ga, or Gd and B represents Ta, Nb, or V).

4. The ultraviolet light absorber according to claim 3, wherein Fe is substituted at 5% and Zn is substituted at 5%.

5. An ultraviolet light absorber produced by substituting Fe at 1% or more to 10% or less into either A or B, or both of them, in a compound expressed by a general formula ABO₄ (where A represents In, Bi, Ga, or Gd and B represents Ta, Nb, or V).

6. The ultraviolet light absorber according to claim 5, wherein Fe is substituted at 10%.

7. An ultraviolet light absorber produced by substituting Zn at 1% or more to 10% or less into either A or B, or both of them, in a compound expressed by a general formula ABO₄ (where A represents In, Bi, Ga, or Gd and B represents Ta, Nb, or V).

8. The ultraviolet light absorber according to claim 7, wherein Zn is substituted at 10%.

9. An ultraviolet light absorber produced by substituting Ni at 1% or more to 10% or less into either A or B, or both of them, in a compound expressed by a general formula ABO₄ (where A represents In, Bi, Ga, or Gd and B represents Ta, Nb, or V).

10. The ultraviolet light absorber according to claim 9, wherein Ni is substituted at 10%.

11. An ultraviolet light absorber produced by substituting In at 1% or more to 10% or less excessively into a compound expressed by a general formula ABO₄ (where A represents In, Bi, Ga, or Gd and B represents Ta, Nb, or V).

12. The ultraviolet light absorber according to claim 11, wherein In is substituted at 10% excessively.

13. A cosmetic material containing the ultraviolet light absorber stated in any one of claim 1 to claim 12.
